# EUROPEAN PATENT APPLICATION

(11) **EP 4 381 946 A1**
(43) Date of publication of application: **12.06.2024**
(21) Application number: 22919276.0
(22) Date of filing: 21.10.2022
(51) Int. Cl.: A01N 63/20, A01N 63/22, A01N 63/27, A01N 63/32, C05F 11/08, C05F 17/20

(54) **AGRICULTURAL SOLUTION COMPRISING BACTERIA, FUNGI AND NATURAL COMPOUNDS HAVING A SOIL-CONDITIONING AND/OR DECOMPACTION ACTION AND A GROWTH-PROMOTING ACTION FOR PLANTS OF AGRICULTURAL IMPORTANCE, INDUSTRIAL PROCESS AND USE THEREOF**

(71) Applicant: Total Biotecnologia Indústria e Comércio S.A., 81460-020 Curitiba - PR (BR)
(72) Inventor: FUKAMI, Josiane, 81460-020 Curitiba / PR (BR); GOMES, Douglas Fabiano, 81460-020 Curitiba / PR (BR); MARCOLINO GOMES, Juliana, 81460-020 Curitiba / PR (BR); DE ASSIS FILHO, Jonas Hipolito, 81460-020 Curitiba - PR (BR)
(74) Representative: Winter, Brandl - Partnerschaft mbB
(86) International application number: PCT/BR2022/050408
(87) International publication number: WO 2024/082037

(57) **Abstract**

The present invention is related to the industrial process and use of a soil conditioning agricultural composition comprising one or more of the species of the genre *Rhodopseudomonas, Pseudomonas, Bacillus* and *Saccharomyces,* as well as the application thereof in cultivations of agricultural interest with the purpose of benefitting physical, physical-chemical and microbiological properties of the soils. In addition to the biological role of each biological component, chitosan is added to the solution which works as a prebiotic supplement benefiting the microbiota established in the environment of application. Surprisingly, the agricultural composition comprising one or more species of the genre *Rhodopseudomonas, Pseudomonas, Bacillus* and *Saccharomyces* acts in the decompaction of soils in diverse cultures, including those that are highly demanding in the initial preparation of the culture. Finally, the agricultural composition promotes the growth and productivity of the plants in a surprising manner.

## Description

### FIELD OF THE INVENTION

The present invention is related to the industrial process of a soil conditioning agricultural composition comprising one or more of the species of the genre *Rhodopseudomonas, Pseudomonas, Bacillus* and *Saccharomyces,* with addition of chitosan, as well as the application thereof in cultivations of agricultural interest, with the purpose of benefitting physical, physical-chemical and microbiological properties of the soil, with emphasis on the unprecedented concept of biological decompaction of agricultural soils.

### BACKGROUND OF THE INVENTION

The search for technologies that ensure greater sustainability to the agricultural activity has been intensified within the last years, since the excessive use of agrochemicals and fertilizers for obtaining high crops in cultivations of agricultural interest became more and more costly. Consequently, there has been a reduction in the quality of the soils and the interruption of the biological processes thereof, which are responsible, mainly, for the mineralization and cycling of the organic nutrients for the plants. Among the several impacts resulting from unsustainable practices, the most expressive ones associated with the degradation of the arable lands are the compacting, erosion, and the reduction of the stocks of organic material, negatively influencing the soil microbiota and macrofauna (LEITE et al., 2003). For this reason, the rational use of the agrochemicals and fertilizers, as well as tools for enhancing soil conservation has become necessary, among which is the application of microorganisms such as fungi and bacteria with specific functional mechanisms that can establish an important role in the conservation and regeneration of arable lands.

*Rhodopseudomonas palustris* known as Purple Non-sulfur Bacteria (PNSB) is widely distributed both in aquatic and terrestrial environments (Gray and Smith, 2005) . The establishment thereof in such distinct environments is extraordinary and is owed to the metabolic versatility thereof, since it grows through any one of the four modes of metabolism that sustain life: photoautotrophic or photosynthetic (energy from light or CO₂), photoheterotrophic (energy from light and carbon from organic compounds), chemoheterotrophic (carbon and energy from organic compounds), and chemoautotrophic (energy from inorganic compounds and CO₂) (Larimer et al., 2005). In addition to the metabolic versatility, these microorganisms synthetize compounds from the secretions of the plant roots, organic matter, and harmful gases, such as hydrogen sulfide, using sunlight and the heat of the soil as energy sources. Synthetized metabolites are directly absorbed by the plants thus promoting their growth (Kim et al. 2004; Higa 2000; Kim and Lee, 2000; Ranjith et al., 2007; Imhoff, 2006). In addition to this characteristic, they can also act as bioremediators with the reduction of the emission of methane gases and heavy metals (Al⁺³, Fe⁺²). However, their soil decompaction action is little known scientifically and the commercial application thereof is non-existent.

The yeasts of the genre *Saccharomyces* can act with direct benefit to the plants by the production of growth promoter metabolites, for example through the synthesis of phytohormones, the solubilization of nutrients, the production of siderophores and the biological control of phytopathogens (Xin et *al.,* 2009, Rosa-Magri et al., 2012, Wang et al., 2009, Rosa et *al.,* 2010).

Apart from the *Rhodopseudomonas* and *Saccharomyces* which work for the benefit of the plants, there also exists the plant growth-promoting bacteria (PGPB) that colonize the roots and/or rhizosphere of the plants and promote their growth. Among the several genres of microorganisms characterized as being PGPB there is emphasis on *Agrobacterium, Allorhizobium, Arthrobacter, Azospirillum, Azotobacter, Bacillus, Bradyrhizobium, Burkholderia, Caulobacter, Chromobacterium, Erwinia, Exiguobacterium, Flavobacterium, Mesorhizobium, Micrococcous, Providencia, Pseudomonas, Rhizobium* and *Serratia* (Yadav et al., 2017; Suman et al., 2015; Suman et al., 2016).

The plants' growth-promoting microorganisms have several action mechanisms, with emphasis on the production of phytohormones as auxins (Bric et al., 1991) and gibberellins (Brown, 1968), the biological fixation of nitrogen (Boddey et al., 1995) phosphorus solubilization (Pikovskaya, 1948), zinc (Fasim et al., 2002) and potassium (Hu and Guo, 2006), the production of the ACC deaminase enzyme (Jacobson et al., 1994), ammonia biosynthesis (Cappucino and Sherman, 1992) siderophores (Schwyn and Neilands, 1987).

The main use of these bacteria for commercial purposes is as plant growth promoters. However, it is still unexplored as regards the purpose of soil conditioner. In this manner, the microorganisms for comprising a commercial product working on the physical, chemical, and microbiological attributes of the soil are carefully chosen so that they perform their functions when applied to several cultures. The main discovery carried out to compose a product with structural and physical purpose is through the selection of the bacteria of the genre *Rhodopseudomonas.* As regards the fertility/chemical and microbiological requirements, they cover the PGPB and yeasts, which, apart from promoting the plants, act on making the nutrients available and hold some mechanisms for the control of soil phytopathogens, thus improving the microbiota existing therein.

Complementing the regenerative and growth promoter action performed by the microorganisms, several natural compounds can be applied to agriculture, in association with the biological products, generating benefits to the entire agricultural system. Among these compounds, chitin, chitosan, polysaccharides, organic acids, algae extracts, are highlighted, among others.

Specifically concerning chitosan, chitin deacetylation product, great potential has been reported for application in crops of agronomic interest. Since it is a biodegradable compound, and biocompatible with beneficial microorganisms, it is extremely well characterized as an important plant protector against biotic and abiotic stress. Additionally, chitosan is described as being a trigger of the plant immune system, guaranteeing greater resistance to diseases (Malerba and Cerana, 2016; 2019).

Microorganism consortiums associated with chitosan in biotechnological formulations applied in agriculture, present a disruptive role in the benefit of the chemical, physical and biological properties of the soil. The potentializing of the function of soil conditioning performed by chitosan can be characterized, as a parallel, with its prebiotic action. That is, the addition of this natural component acts as a complex nutrient source, stimulating the microbiota of the soil to exert their respective biological roles, strongly contributing to the reestablishment of the optimal conditions of the soil, regenerating degraded systems.

The industrial production, see detailed technical description, of the agricultural composition goes through a complex process which ensures the cellular concentration of all the microorganisms cultivated at their respective guaranteed levels. This process involves specific parameters such as growth temperature, air volume, agitation, and pressure (related to the oxygen rate dissolved in the cultivation medium).

Thus, the industrial production with fundamental fermentative parameters for producing different species of *Rhodopseudomonas, Pseudomonas, Bacillus* and *Saccharomyces* that ensure the stability and cellular viability for the agricultural application as a soil conditioning agricultural composition is an important innovative tool, unparalleled in commercial scale for improving conservation of arable land.

### REFERENCES

Boddey, R.; De Oliveira, O.; Urquiaga, S.; Reis, V.; De Olivares, F. et al. Biological nitrogen fixation associated with sugar cane and rice: contributions and prospects for improvement. Plant Soil. v. 174, n. 1-2, p. 195-209, 1995.
Brie, J.M.; Bostock, R.M.; Silverstone, S.E. Rapid in situ assay for indoleacetic acid production by bacteria immobilized on a nitrocellulose membrane. Appl Environ Microbiol. v. 57, n. 2, p. 535-538, 1991.
Brown, Peter H.; HO, Tuan-Hua David. Barley aleurone layers secrete a nuclease in response to gibberellic acid: purification and partial characterization of the associated ribonuclease, deoxyribonuclease, and 3'-nucleotidase activities. Plant Physiology, v. 82, n. 3, p. 801-806, 1986.
Cappucino, J.C.; Sherman, N. Nitrogen Cycle. In: Microbiology: A Laboratory Manual. (4th edn), Benjamin/Cumming Pub Co, New York, USA, p.311-312, 1992.
Fasim, F.; Ahmed, N; Parsons, R.; Gadd, G.M. Solubilization of zinc salts by a bacterium isolated from the air environment of a tannery. FEMS Microbiol Lett. v. 213, n. 1, p. 1-6, 2002.
Hu X, Chen J, Guo J. Two Phosphate- and Potassiumsolubilizing Bacteria Isolated from Tianmu Mountain, Zhejiang, China. World J Microbiol Biotechnol. v. 22, n. 9, p. 983-990, 2006.
Jacobson, C.B.; Pasternak, J.; Glick, B.R. Partial purification, and characterization of 1-aminocyclopropane-1-carboxylate deaminase from the plant growth promoting rhizobacterium Pseudomonas putida GR12-2. Can J Microbiol v. 40, n. 12, 1019-1025, 1994.
Pikovskaya, R. Mobilization of phosphorus in soil in connection with vital activity of some microbial species. Mikrobiologiya. v. 17, p. 362- 370, 1948.
Schwyn, B.; Neilands, J. Universal chemical assay for the detection and determination of siderophores. Anal Biochem v. 160, n. 1, p. 47-56, 1987.
Suman, A.; Verma, P.; Yadav, N.A.; Saxena, A.K. Bioprospecting for extracellular hydrolytic enzymes from culturable thermotolerant bacteria isolated from Manikaran thermal springs. Res J Biotechnol. v. 10, p. 33-42, 2015.
Suman, A; Verma, P.; Yadav, A.N.; Srinivasamurthy, R.; Singh, A.; Prasanna, R. Development of hydrogel-based bio-inoculant formulations and their impact on plant biometric parameters of wheat (Triticum aestivum L.). Int J Curr Microbiol Appl Sci. v. 5, n. 3, p. 890-901, 2016.
Yadav, A.N.; Verma, P.; Kumar, V.; Sachan, S.G.; Saxena, A.K. Extreme Cold Environments: A Suitable Niche for Selection of Novel Psychrotrophic Microbes for Biotechnological Applications. Adv Biotechnol Microbiol. v. 2, n. 2, p. 1-4, 2017.
Imhoff, J. C. F. The phototrophic alphaproteobacteria, p. 41-64. In M. Dworkin, S. Falkow, E. Rosenberg, K.H. Schleifer, and E. Stackebrandt (ed.), The Prokaryotes: A Handbook on the Biology of Bacteria. Proteobacteria: Alpha and Beta Subclass. Springer-Verlag, New York. 2006.
Kim, M. K.; Choi, K. M.; Yin, C. R. Odorous swine wastewater treatment by purple non-sulfur Bacteria, Rhodopseudomonas palustris, isolated from eutrophicated ponds. Biotechnol Lett v. 26, p. 819-822, 2004.
Kim, J. K.; Lee, B. K. Mass production of Rhodopseudomonas palustris as diet for aquaculture. Aquacult Eng. v. 23, p. 281-293, 2000.
Ranjith, N. K., Sasikala, C., Ramana, C. V. Catabolism of l-phenylalanine and l-tyrosine by Rhodobacter sphaeroides OU5 occurs through 3, 4-dihydroxyphenylalanine. Res Microbiol. v. 158, p. 506-511, 2007.
Gray, E.J.; Smith, D.L. Intracellular and extracellular PGPR: commonalities and distinctions in the plant-bacterium signaling processes. Soil Biol Biochem. V.37, p. 395-412, 2005.
Larimer, F. W.; Chain, P.; Hauser, L.; Lamerdin, S.; Malfatti, S.; Do, L.; Land, M.L.; Pelletier, D. A.; Beatty, J. T.; Lang, A. S.; Tabita, F. R.; Gibson, J. L.; Hanson, T. E.; Bobst, C.; Torres, J. L.T.; Peres, C.; Harrison, F. H.; Gibson, J.; Harwood, C. S. Complete genome sequence of the metabolically versatile photosynthetic bacterium Rhodopseudomonas palustris. Nature biotechnology, v. 22, n. 1, p. 55-61, 2004.
Leite, L. F. C.; Mendonça, E. S.; Machado, P. L. O. A.; Matos, E. S. Total C and N storage and organic C pools of a Red-Yellow Podzolic under conventional and no tillage at the Atlantic Forest Zone, Southeastern Brazil. Austr. J. Soil Res., v.41, p.717-730, 2003.
Malerba, M.; Cerana, R. Chitosan effects on plant systems. Int. J. Mol. Sci. v.17, p. 996-1010, 2016.
Malerba, M.; Cerana, R. Recent applications of chitin- and chitosan-based polymers in plants. Polymers. v.11, p.839-847, 2019.
Ralisch, R., Debiasi, H., Franchini, J. C., Tomazi, M., Hernani, L. C., Melo, A. da S., Santi, A. L. da S., DE Bona, F. D. O diagnóstico rápido da estrutura do solo - DRES. Embrapa - Empresa Brasileira de Pesquisa Agropecuária. 2017. (Documento 390).
Rosa, M. M., Tauk-Tornisielo, S. M., Rampazzo, P. E., Ceccatoantonini, S. R. Evaluation of the biological control by yeast Torulaspora globosa against Colletotrichum sublineolum in sorghum. World Journal of Microbiology and Biotechnology, v.26, p.1491-1502, 2010.
Rosa-Magri, M. M, Avansini, S. H., Lopes-Assad, M. L., Tauk-Tornisielo, S. M., Ceccato-Antonini, S. R. Release of potassium from rock powder by the yeast Torulaspora globosa. Brazilian Archives of Biology and Technology, v.55, 2012.
Silva, G. O. da; Lopes, C. A. Sistema de Produçäo de Batatas Embrapa Hortaliças (INFOTECA-E), 2015.
Wang, W. L., Chi, Z. M., Li, J., Wang, X. H. Siderophore production by the marine derived Aureobasidium pullulans and its antimicrobial activity. Bioresource Technology, v.100, p.2639-2641, 2009.
Xin, G.; Glawe, D.; Doty, S. L. Characterization of three endophytic, indole-3-acetic acid-producing tests occurring in Populus trees. British Mycological Society, Mycological Research, v.113, p.973 - 980, 2009.

### SUMMARY OF THE INVENTION

The present invention uses microorganisms of the genre Rhodopseudomonas, *Pseudomonas, Bacillus* and *Saccharomyces,* as well as chitosan, chitin derived compound which, when associated, are able to improve the physical, physical-chemical, and microbiological properties of the soil, promoting plant growth, and resulting in greater productivity in the employed cultures.

The present invention allows industrial scale production, replicable and commercially viable of a biological soil decompactor.

The applicability of the invention is provided, mainly, for application in cultures of agricultural interest, whereby it can be used in different cultures, types of soils and product concentrations, as well as different application times.

The present invention teaches that, surprisingly, it is possible to develop a complex biotechnological solution (in industrial scale) containing one or more species of *Rhodopseudomonas, Pseudomonas, Bacillus* and *Saccharomyces* as soil conditioners.

The present invention also teaches that the addition of chitosan to the microorganism consortium enhances in a surprising manner the soil conditioning effect of the biotechnological solution.

Advantageously, the present invention allows obtaining an agricultural composition that can be applied to the field as a tool for soil conservation in several cultures of agricultural interest, such as soybean, corn, wheat, rice, pastures, fruit & vegetables, among others.

As will be understood by a person skilled in the art, the present invention provides additional parameters for the method of producing an agricultural composition formed by one or more species of *Rhodopseudomonas, Pseudomonas, Bacillus* and *Saccharomyces* fermented in industrial scale, demonstrating the necessary parameters for the induction of metabolites, such as pressure, temperature, oxygenation (air volume and agitation) induction, and culture means, enabling obtaining a biotechnological product.

In a first embodiment, the present invention provides a process for producing an agricultural composition comprising the steps of:
(a) fermentation of microorganisms comprising seven species, namely *Rhodopseudomonas palustris, Pseudomonas fluorescens, Bacillus subtilis, B. amyloliquefaciens, B. pumilus, B. licheniformis* and *Saccharomyces boulardii* capable of improving the physical, physical-chemical, and microbiological properties of the soil, promoting plant growth, through the specific formulation for each microorganism during the industrial process; and
(b) formulation of a biotechnological product comprised by mixture of bacteria, in concentrations from 1,0 × 10⁶ to 1,0 × 10⁹ UFC/mL, and chitosan, in a proportion from 0,1 to 5% (p/v), in a technical solution which allows application in agriculture as a soil conservation tool.

In a surprising manner, the present invention has as its preferred embodiment the ability of improving the physical, physical-chemical, and microbiological properties of the soil.

In a secondary embodiment, unexpectedly, the present invention is able to promote the growth of plants and increase productivity of cultures of agronomic interest.

### BRIEF DESCRIPTION OF THE FIGURES

For a more complete understanding of the invention, reference must now be made to the embodiments of the invention illustrated in more detail in the attached figures and described by means of the embodiments of the invention.

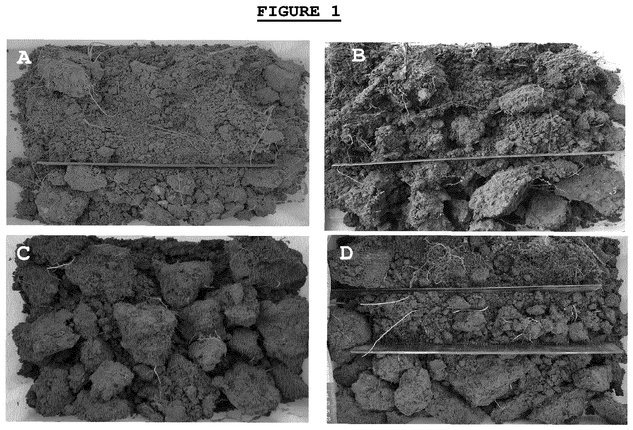

Figure 1 illustrates the Soil Structural Quality Index (IQES) by the DRES method at the time of Harvest of the cabbage cultivation 75 days after transplantation. A. Control; B. Control 100% N; C. 50% N + *Rhodopseudomonas palustris* + *Saccharomyces boulardii* at a dosage of 2L/ha; D. 50% N + *Rhodopseudomonas palustris* + *Saccharomyces boulardii* at a dosage of 3L/ha.

### DETAILED DESCRIPTION OF THE INVENTION

In a preferred embodiment, according to the present invention, the fermentation (step (a)) of the species of *Rhodopseudomonas, Pseudomonas, Bacillus* and *Saccharomyces* by batch occurs for approximately 24 - 168 hours.

In a preferred embodiment, the method of the present invention comprises the sequential expansion (scaling) of the culture of *Rhodopseudomonas, Pseudomonas, Bacillus* and *Saccharomyces* for inoculation of the fermentation culture. Preferably, the sequential expansion starts at 100 mL volumes, which serves as inoculum of the fermentation culture. Preferably the sequential expansion starts at 100 mL volumes, which serves as inoculum for 1 L. This, in turn, is inoculated in 10 L, which are then inoculated in two flasks in 180 L tanks and, finally, are transferred to reactors containing 2.000 L.

In a preferred embodiment, the species of *Rhodopseudomonas, Pseudomonas, Bacillus* and *Saccharomyces* are expanded in flasks of 100 mL by incubation in orbital agitator from 80 rpm to 200 rpm. The incubation time is preferably from 8 to 48 hours. Preferably, the species of *Rhodopseudomonas, Pseudomonas* and *Saccharomyces* are cultivated in flasks of about 1 L of culture medium by incubation in orbital agitator from 80 rpm to 200 rpm. Preferably, the species of *Bacillus* are then cultivated in stainless steel flasks containing about 1 L of culture medium. The incubation time is preferably from 8 to about 48 hours with air flow at about 0,25 Nm³/h to about 1,0 Nm³/h (=4,16 - 16, 67 vvm) .

In a preferred embodiment, the air flow of the stainless steel flasks containing about 10 L is from about 0,25 to about 1,5 Nm³/h (= 0,41 - 2,5 vvm), and the incubation time is preferably from about 8 hours to about 48 hours.

In a preferred embodiment, the air flow of the stainless steel flasks containing 10 L for the cultivation containing the species of *Rhodopseudomonas, Pseudomonas, Bacillus* and *Saccharomyces* is from 0,25 to 1,5 Nm³/h (= 0,41 - 2,5 vvm), and the incubation time is preferably from about 8 hours to about 48 hours.

In a preferred embodiment, the incubation temperature for multiplication of the species of *Rhodopseudomonas, Pseudomonas, Bacillus* and *Saccharomyces* according to the present invention is from 22 °C to 38 °C.

In a preferred embodiment, the species of *Rhodopseudomonas, Pseudomonas, Bacillus* and *Saccharomyces* are inoculated separately in the scaling process up to 180 L and mixed in the fermenters of 2.000 L as described for the present invention. For this purpose, in a preferred embodiment, after the cultivation of the *Rhodopseudomonas, Pseudomonas* and *Saccharomyces* in 1 L flasks of culture medium and *Bacillus* in stainless steel flasks containing 1 L medium, next they are inoculated in two stainless steel flasks containing 10 L of culture medium, and then transferred to tanks containing 180 L of the specific culture medium for each microorganism. For the *Bacillus,* the culture medium used up to the scales of 10 L are equal for all species.

Preferably, the cultivation media for the scales up to 180 L are specific for each genre of microorganism. The air flow is, preferably, from 1,0 to 15,0 Nm³/h (= 0,16 - 1,25 vvm) and the incubation occurs for 24 to 168 hours.

In a preferred embodiment, the step of mixture of the species of *Rhodopseudomonas, Pseudomonas, Bacillus* and *Saccharomyces* is conducted at temperature from 22 °C to 38 °C. The air flow is preferably from 1,0 Nm³/h to 2,5 Nm³/h (= 0,0085 - 0,021 vvm). The pressure is preferably from 0,5 to 1,2 kgf/cm³. The agitation is preferably from 40 hz to 45 hz.

In a preferred embodiment, after the mixture, the concentrations of each constituent species of the present invention can vary in the range comprising 1,0 × 10⁶ to 1,0 × 10⁹ UFC/mL.

### EXAMPLES

### EXAMPLE 1 - CULTURE SCALING

The different species of *Rhodopseudomonas, Pseudomonas, Saccharomyces* and *Bacillus* are inoculated separately in flasks containing 100 mL of culture medium as described in Tables 1 and 2, being incubated in orbital agitator at about 80-200 rpm, at 22-38 °C for approximately 8-48 hours. The 100 mL inoculums of *Rhodopseudomonas, Pseudomonas* and *Saccharomyces* are then transferred to flasks containing 1L of culture medium, being incubated in orbital agitator at 80 rpm to 200 rpm, at a temperature of 22-38 °C for approximately 8-48 hours. After the incubation period, the cultivations of the species of *Rhodopseudomonas, Pseudomonas* and *Saccharomyces* are inoculated in stainless steel flasks containing 10 L of specific culture medium for each microorganism as described in table 1 and incubated for approximately 18 - 96 hours, with air flow 0,25 - 1,5 Nm³/h (= 0,41 - 2,5 vvm) and temperature varying from 22 to 38 °C. For the *Bacillus,* the flasks containing 100 mL of cultivation are then inoculated in stainless steel flasks containing 1 L of medium, next they are inoculated in two stainless steel flasks containing 10 L of culture medium as described in Table 2 and incubated for approximately 18 - 96 hours, with air flow 0,25 - 1,5 Nm³/h (= 0,41 - 2,5 vvm) and temperature varying between 22-38 °C.

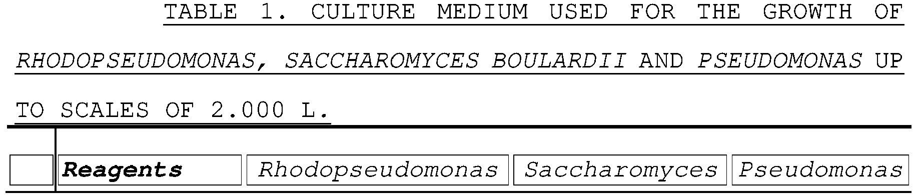

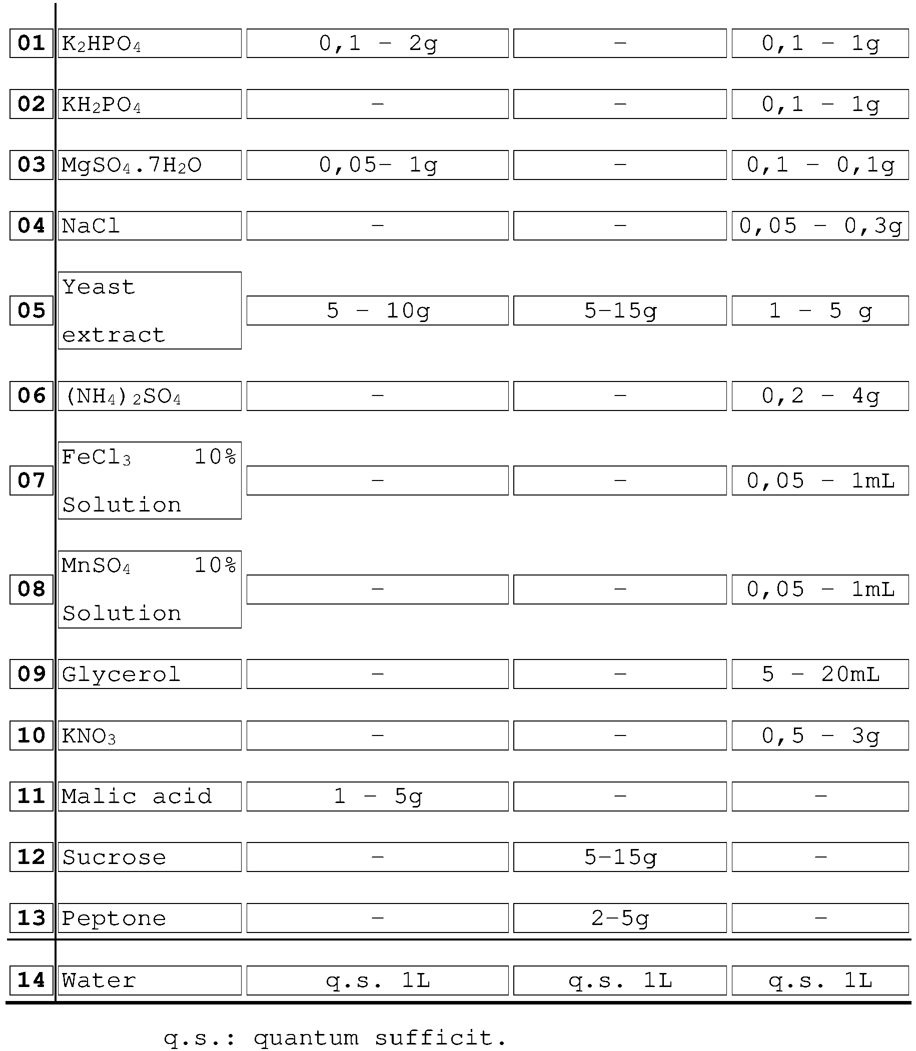

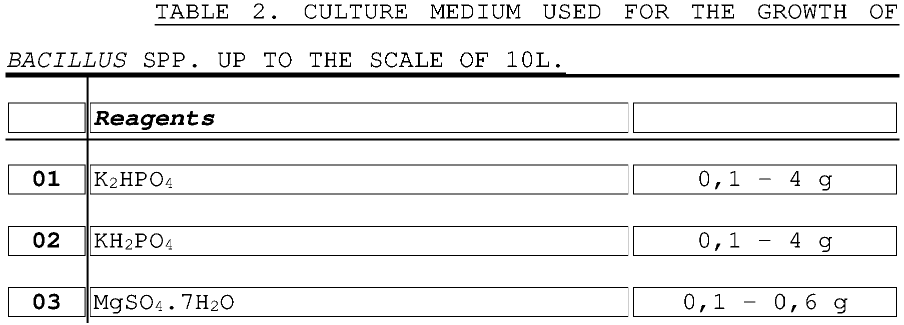

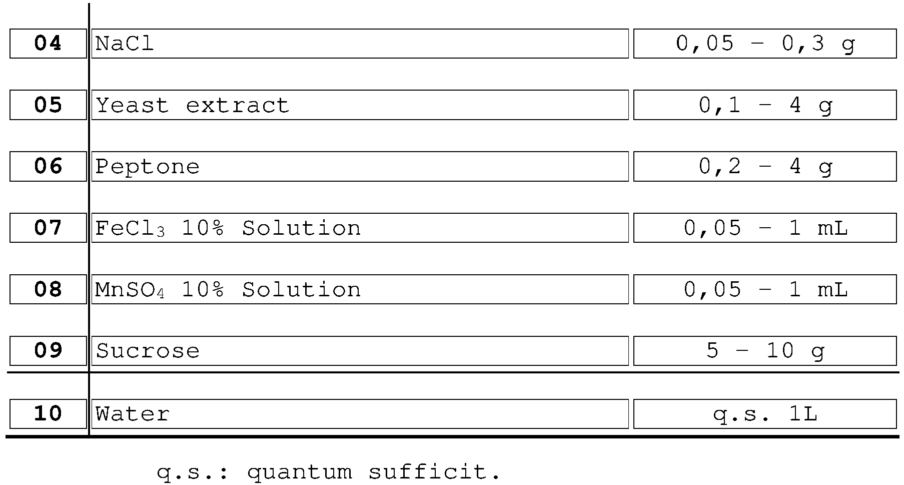

After this period has lapsed, each culture containing two stainless steel flasks with 10 L of the culture medium are inoculated in a tank containing about 180 L of the specific culture medium for each microorganism, the specific culture medium for *Rhodopseudomonas, Pseudomonas* and *Saccharomyces* and in Table 3 the specific culture medium for the different species of *Bacillus.* After the inoculation the microorganisms are incubated for approximately 24 - 168 hours, with air flow 3,0 - 10,0 Nm³/h (= 0,25 - 0,83 vvm) and temperature varying from 22 - 38 °C.

For the growth process in 2.000 L fermentors the sterilization process of 1.800 L of culture medium is carried out for approximately 60 to 120 minutes, at a temperature of approximately 121 °C to approximately 130 °C. Preferably, the sterilization is carried out at a pressure of approximately 1,0 - 2,0 Kgf/cm². After the period of sterilization and cooling, the tank containing *Rhodopseudomonas, Pseudomonas,* four species of *Bacillus* and *Saccharomyces* is then inoculated to the respective 2.000 L fermentors containing the specific culture medium for each microorganism and the incubation time is preferably from 24 to 72 hours, at a temperature of 22°C - 38°C. The air flow is preferably 1,0 Nm³/h to 2,5 Nm³/h (= 0,0085 - 0,021 vvm) . The pressure is preferably from 1,0 to 2,0 kgf/cm³. Agitation is preferably from 40 hz to 45 hz. For the growth and sporulation of the *Bacillus* in 2.000 L fermentor there is added a stainless steel flask containing about 10 L of the solution of endospore forming salts according to Table 4.

The scaling process for *Rhodopseudomonas* and *Saccharomyces* continues, preferably, in 10.000 L fermentor. For this scale, the sterilization process uses 6.000 L of the formulation described in Table 5, carried out for approximately 60 to 120 minutes, at a temperature of approximately 121 °C to approximately 130 °C. Preferably, the sterilization is carried out at a pressure of approximately 1,0 - 2,0 Kgf/cm². After the sterilization and cooling period, the 2.000 L fermentor containing *Rhodopseudomonas* is then inoculated to the 10.000 L fermentor and the incubation time is preferably from 24 to 72 hours at a temperature of 22°C -38°C. The air flow is preferably 5,0 Nm³/h to 12,5 Nm³/h (= 0,0085 - 0,021 vvm). The pressure is preferably from 1,0 to 2,0 kgf/cm³. The agitation is preferably from 40 hz to 45 hz.

Preferably, after the incubation time of the *Rhodopseudomonas,* there are added 2.000 L of the cultivation of *Saccharomyes* with incubation time preferably from 24 to 72 hours and temperature between 22°C-38°C. The air flow is preferably 5,0 Nm³/h to 12,5 Nm³/h (= 0,0085 - 0,021 vvm). The pressure is preferably from 1,0 to 2,0 kgf/cm³. The agitation is preferably from 40 hz to 45 hz.

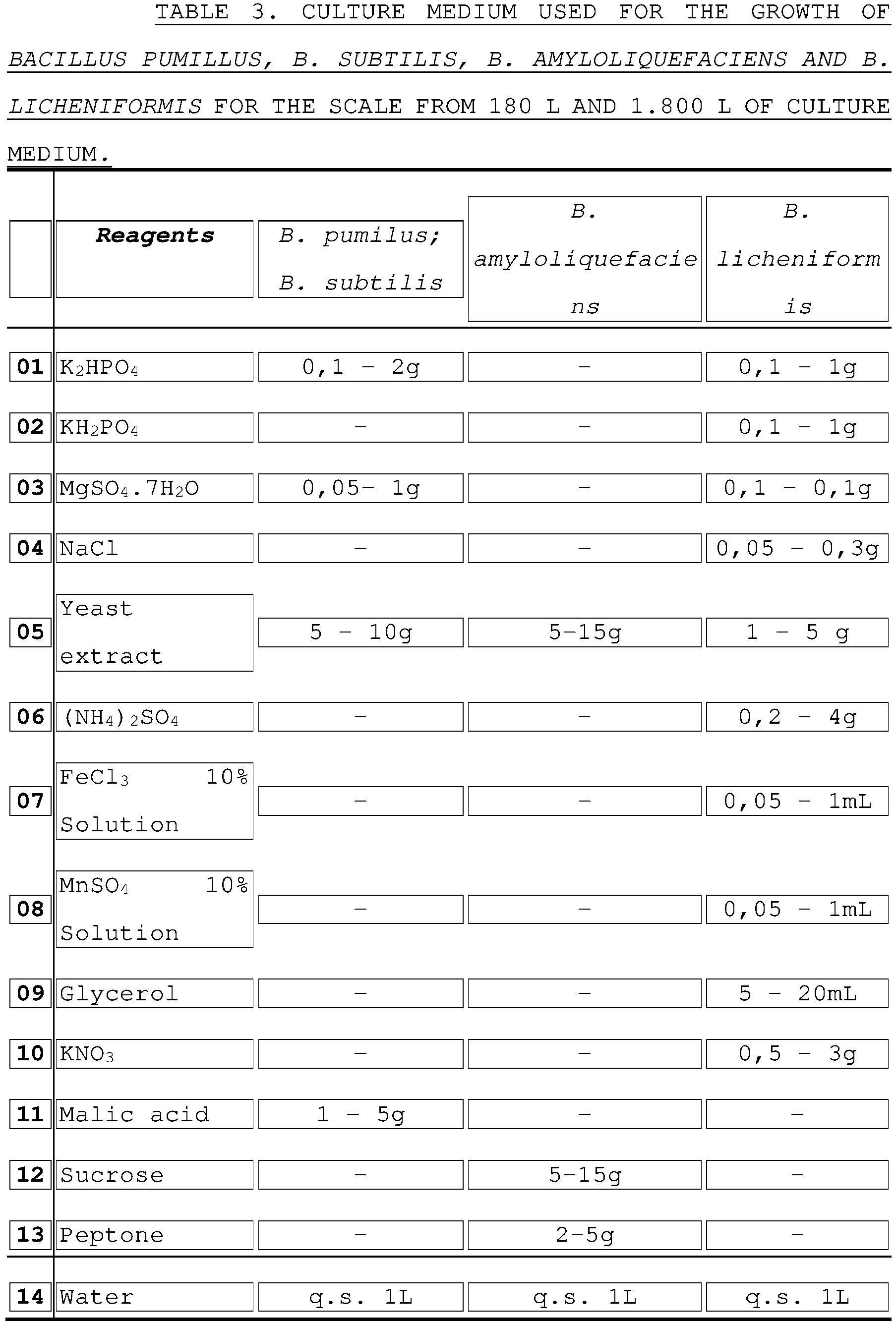

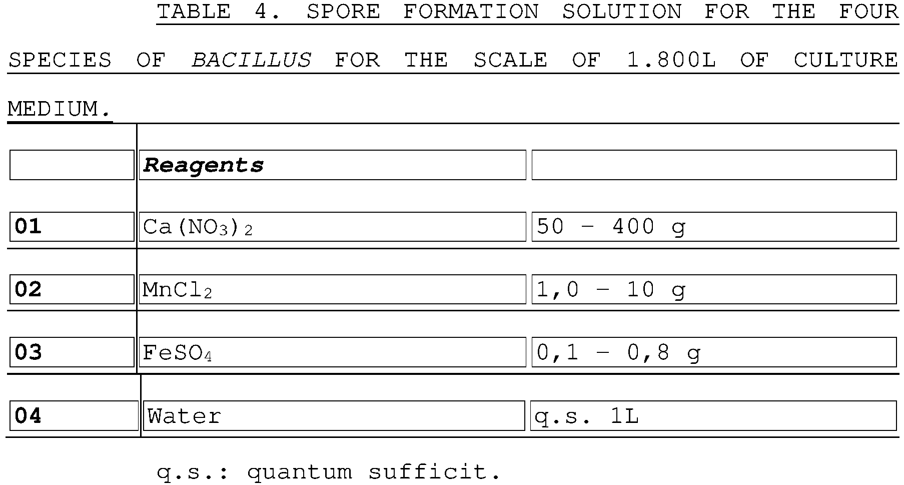

### EXAMPLE 2 - MIXTURE OF THE MICROORGANISMS AND CHITOSAN IN BIOREACTOR

The mixture of the species of *Rhodopseudomonas* and *Pseudomonas, Saccharomyces,* the four species of *Bacillus* and the chitosan occurs in a 20.000 L mixer in a process from 60 to 180 minutes.

Preferably, the quantity of chitosan that comprises the product is from 0,1 to 5 % of the final volume of the product.

Preferably, the product is filled in rigid vials, package in which the product is stored.

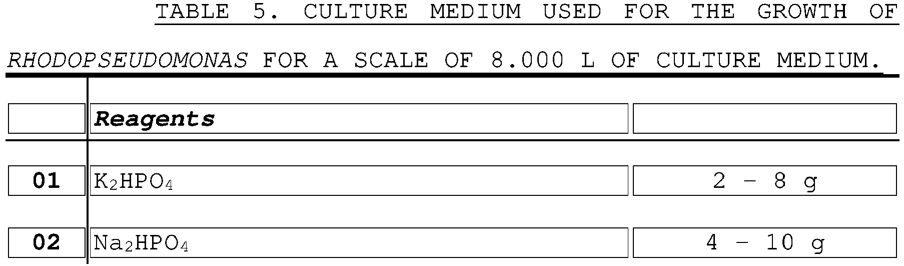

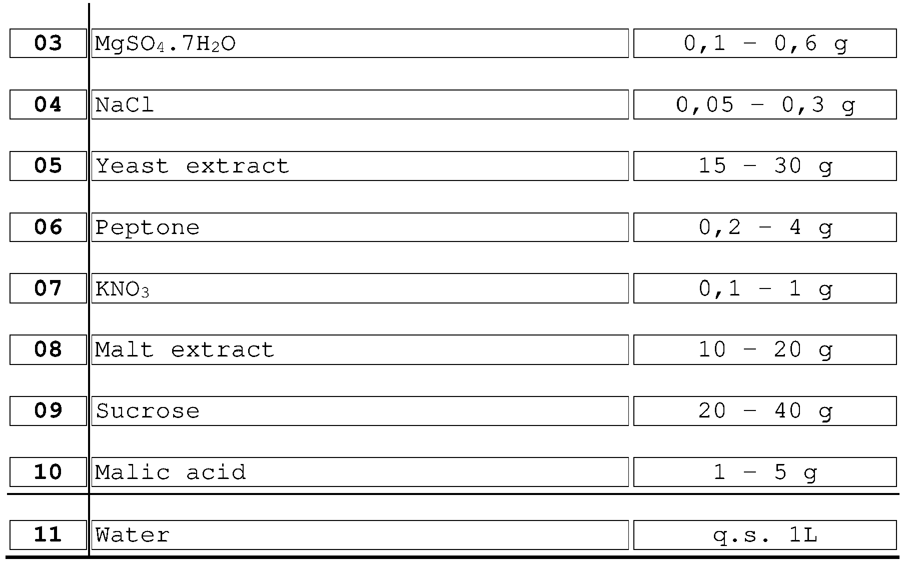

### EXAMPLE 3 - THE COMBINATION OF MICROORGANISMS AND CHITOSAN AS A CONSERVATION TOOL FOR SOILS IN CROPS OF AGRONOMIC IMPORTANCE.

There were carried out field trials in different highly exacting cultures related to soil structuring and soil fertility, particularly for cultures such as potatoes, since it is necessary to carry out an initial preparation of the soil as regards the decompaction of the first 30 cm, below this the energy and productive cost turns out costly (Silva & Lopes, 2015). Thus, to help in soil decompaction the use of microorganisms which act in this part can become a highly profitable tool, as observed in table 6, wherein the total number of tubers when the microorganisms are added, especially the *Rhodopseudomonas* and *Saccharomyces* (T2) presented statistical difference when compared to the non-inoculated control, providing a 200 increase in the productivity of the culture. In the same manner, when the species of *Bacillus* (T3) are added, we have an increase of 11% when compared to the non-inoculated control. Another important point to be mentioned is that this increase was in the number of special tubers (tubers larger than 35 mm), a characteristic pursued by the farmers aggregating greater productive profitability. The increase of the components of the production in the same way resulted in greater culture productivity in the same treatments as evaluated in table 7, wherein the treatments with *Rhodopseudomonas* and *Saccharomyces* (T2) provided an increase of 21% in the productivity and the addition of the species of *Bacillus* (T4) provided an increase of 16% in the productivity of the culture. Another important component for the culture is the increase in the percentage of soluble solids, since they can be destined to the processing in the industry, improving the quality of French fries. The application of microorganisms did not significantly affect this parameter, whereby a tendency to increase can be observed.

The addition of *Pseudomonas* with the *Rhodopseudomonas* and *Saccharomyces* also resulted in higher production of special tuber with a 20% increase when compared to the non-inoculated chemical control (Table 8). It is important to highlight that the treatments that received the chemical control associated with the biological one did not reach the productivity levels of the one that used only the biological one, particularly for top quality tubers (25- and 35-mm diameter).

**TABLE 6. COMPONENTS OF THE PRODUCTION OF THE CULTURE OF CULTIVAR POTATO ATLANTIC TYPE III INOCULATED VIA FURROW AND HEAP OF DIFFERENT MICROORGANISMS.**

| **Treatment** | **#Plants** | **#Stems** | **#Special Tubers** | **Tubs 1^{a}** | **#Tubs 2^{a}** | **#Tubs Totals** |
|---|---|---|---|---|---|---|
| T1. Control | 37,4 a | 50,2 a | 114,2 b | 5,8 a | 0,2 a | 120,2 b |
| T2. *Rp+Sb* | 36,8 a | 48 ab | 138,2 a | 5,6 a | 0,8 a | 144,6 a |
| T3. *Bs+Bp+Ba* | 36,4 a | 43,6 b | 124 ab | 8,8 a | 0,2 a | 133 ab |
| T4. *Rp+Sb+Bs+Bp +Ba* | 36,2 a | 46,8 ab | 126,8 ab | 6,8 a | 0,2 a | 133,8 ab |
| **C.V.(%)** | **6,23** | **7,21** | **9,65** | **41,04** | **173,0 1** | **10,24** |

Completely randomized design (CRD). Means (5 repetitions) followed by the same letter in the same column do not present statistical difference between the treatments by the Duncan test (p≤0,05). Rp: *Rhodopseudomonas palustris;* Sb: *Saccharomyces boulardii;* Bs: *Bacillus subtilis;* Bp: *B. pumilus;* Ba: *B. amyloliquefaciens.*

**TABLE 7. PRODUCTIVITY OF THE CULTURE OF CULTIVAR POTATO ATLANTIC TYPE III INOCULATED VIA FURROW AND HEAP OF DIFFERENT MICROORGANISMS.**

| **Treatment** | **Special Productio n (Kg/ha)** | **Product ion 1^{a} (Kg/ha)** | **Producti on 2^{a} (Kg/ha)** | **Total productivit y (Kg/ha)** | **% Soluble Solid** |
|---|---|---|---|---|---|
| T1. Control | 23.970 b | 245 a | 2,6 a | 24.217,6 b | 17,18 b |
| T2. *Rp+Sb* | 29.170 a | 220 a | 10,2 a | 29.400,2 a | 17,3 b |
| T3. *Bs+Bp+Ba* | 27.095 ab | 360 a | 2,6 a | 27.457,6 ab | 17,52 a |
| T4 . *Rp+Sb+Bs+ Bp+Ba* | 27.860 ab | 272,6 a | 2,6 a | 28.135,2 ab | 17,16 b |
| **C.V.(%)** | **12,03** | **41,49** | **171,76** | **12,07** | **0,83** |

Completely randomized design (CRD). Means (5 repetitions) followed by the same letter in the same column do not present statistical difference between the treatments by the Duncan test (p^0,05). Rp: *Rhodopseudomonas palustris;* Sb: *Saccharomyces boulardii;* Bs: *Bacillus subtilis;* Bp: *B. pumilus;* Ba: *B. amyloliquefaciens.*

**TABLE 8. PRODUCTIVITY OF CULTURE OF CULTIVAR ATLANTIC POTATO INOCULATED VIA FURROW WITH DIFFERENT MICROORGANISMS.**

| **Treatment** | **Special Production (Kg/ha)** | **Product ion 1^{a} (Kg/ha)** | **Product ion 2^{a} (Kg/ha)** | **Total productivi ty (Kg/ha)** | **% Soluble Solids** |
|---|---|---|---|---|---|
| T1. Chemical control | 16.925 | 550 | 69 | 17.544 | 16, 8 |
| T2. *Rp+Sb+Pf* | 19.513 | 263 | 56 | 19.831 | 17,3 |
| T3. Chemical control + *Rp+Sb+Pf* | 19.675 | 775 | 119 | 20.569 | 17,3 |

| | | | | | |
|---|---|---|---|---|---|
| Rp: *Rhodopseudomonas palustris;* Sb: *Saccharomyces boulardii;* Pf: *Pseudomonas fluorescens.* | | | | | |

Additionally, there were carried out trials with the culture of cabbage (*Brassica oleracea* var. *capitata*) with application of the biological *Rhodopseudomonas* and *Saccharomyces* at the time of transplantation of seedlings with 2 and 3 L/ha and reduction of 50% of nitrogen (N), a negative control and a control with 100% N. Concerning the production parameters, the treatments with reduction of 50% N independent of the dosage applied did not present statistical difference when compared to the control with 100% N (Table 9), the results suggest the improvement in the fertility of the soil and that the use of the biological can replace the reduced amount of N for the culture. And the increase in the dosage of the biological did not present a greater gain, therefore, the dose of 2 L/ ha would suffice to meet the nutritive demand for the culture.

Moreover, before the harvest there were carried out two vigor assessments 22 and 37 days after the transplantation (DAT), with point attribution according to the ten points scale, whereby 1 point is awarded to the worst plants, with very reduced vigor, without production, sharp defoliation, compromised nutrition, no possibility of recovery; and 10 points to the plants with excellent vigor, more leafy and with sharp growth, reaching the expected production for the cultivar. In both, the days evaluated, all the treatments with the biological, and the control 100% N did not differ statistically (Table 10), demonstrating once more the potential of the biological in the quality of the culture.

Finally, the structuring of the soil was qualified during harvest based on visually detected characteristics in samples of the first 25 cm by the DRES method (Quick Diagnosis of Soil Structure), wherein a punctuation from 1 to 6 is attributed, where "6" is indicative of the best structural condition and "1" represents the totally degraded soil by the Soil Structural Quality Index (IQES) (Ralisch et al., 2017). Concerning the IQES the treatment with 2L/ha of biological and 50% N presented statistical difference when compared to all the treatments evaluated, particularly the control with 100% N (Table 10), presenting better structural condition of the soil as observed in Figure 1. These results corroborated the potential of these microorganisms particularly the *Rhodopseudomonas palustris* and *Saccharomyces boulardii* as soil conditioner, demonstrating the effect of soil decompaction, improvement in the structural quality of the soil even in a short period and, particularly in cultures which demand stringent requirements in the preparation of the soil before cultivation as is the case of the vegetables.

**TABLE 9. HEAD DIAMETER, COMPACTION, HEAD WEIGHT, DRY MASS AND RELATIVE GAIN INOCULATED DURING TRANSPLANTATION WITH DIFFERENT RHODOPSEUDOMONAS AND SACCHAROMYCES IN CULTURE OF CABBAGE, HYBRID ASTRUS PLUS.**

| **Treatment** | **Head diamete r¹ (cm)** | **Head compaction¹ (points 0-5)** | **Head weight¹ (Kg)** | **Dry mass (g)** | **Relativ e gain (%)** |
|---|---|---|---|---|---|
| T1. Control | 14,23 b | 3,83 a | 0,93 b | 72,00 b | 0,0 |
| T2. 100% N | 22,10 a | 4,22 a | 2,94 a | 172,80 a | 215,2 |
| T3. 50%N+*Rp*+*Sb* (2L/ha) | 20,27 a | 4,30 a | 2,32 a | 146,01 a | 149,0 |
| T4. 50%N+*Rp*+*Sb* (3L/ha) | 20,54 a | 4,28 a | 2,41 a | 164,20 a | 158,4 |
| CV (%)² | 12, 57 | 11,04 | 9, 47 | 13, 34 | |

| | | | | | |
|---|---|---|---|---|---|
| 1. Means followed by the same letter in the column, do not present statistical different between the treatments by Duncan test (p≤0,05). 2. Coefficient of variation in percentage. Rp: *Rhodopseudomonas palustris;* Sb: *Saccharomyces boulardii.* | | | | | |

**TABLE 10. VIGOR AND SOIL STRUCTURAL QUALITY INDEX (IQES) BY THE DRES METHOD INOCULATED DURING TRANSPLANTATION WITH DIFFERENT MICROORGANISMS IN THE CULTURE OF CABBAGE, HYBRID ASTRUS PLUS.**

| **Treatment** | **Vigor¹(Points 1 to 10)** | | **DRES Method (IQES)** |
|---|---|---|---|
| | **22 DAT²** | **37 DAT** | |
| T1. Control | 4,50 b | 3,67 b | 1,62 b |
| T2. 100% N | 7,00a | 7,83 a | 1,67 b |
| T3. 50%N+*Rp*+*Sb* (2L/ha) | 6,50 a | 6,83 a | 3,65 a |
| T4. 50%N+*Rp*+*Sb* (3L/ha) | 7,33 a | 8,00 a | 2,23 b |
| **CV (%)³** | **8,37** | **10,79** | **9,43** |

| | | | |
|---|---|---|---|
| 1. Means followed by the same letter in the column, do not present statistical different between the treatments by Duncan test (p≤0,05). 2. DAT: Days after Transplantation. 3. Coefficient of variation in percentage. Rp: *Rhodopseudomonas palustris;* Sb: *Saccharomyces boulardii* | | | |

The soil conditioning agricultural composition was also assessed for large cultivations such as beans (*Phaseolus vulgaris*) which do not require such an exacting soil preparation as the vegetables, and in the same manner, the larger doses of 2 and 3L/ha of the biological and 50%N provided an increase of 332,6 and 453,6 kg/ha in productivity relative to the treatment with 1005 N, respectively (Table 11). This increase is mainly due to the weight of the grains, since the number of pods per plant and grains per pod did not increase with the application of the agricultural composition. The application of the biologicals via furrow, apart from promoting more productivity to the plantation, once more provided better IQES in the two largest doses applied. These results reinforce the soil conditioner role and the selection directed to the microorganisms provide the farmer the ability to produce with greater profitability and sustainability.

**TABLE 11. AERIAL PART DRY MASS (MSPA), PRODUCTIVITY, WEIGHT OF 1000 GRAINS, NUMBER OF PODS PER PLANT, NUMBER OF GRAINS PER POD AND DRES IN DIFFERENT TREATMENTS OF THE SOIL CONDITIONING AGRICULTURAL COMPOSITION IN BEAN CULTURE.**

| **Treatment** | **MSPA (g)** | **Product ion (kg/ha)** | **1000 grains (g)** | **pods/ plant** | **grain s/ pod** | **DRES (IQES )** |
|---|---|---|---|---|---|---|
| T1: Non-inoculated control | 7,63 ab | 2008,8 c | 184,2 c | 9,78ns 2 | 5,95n s2 | 1,63 b |
| T2: 50% N | 6,34 b | 2133,0 de | 187,7 be | 9,2 | 6,21 | 1,75 ab |
| T3: 100% N | 7,94 a | 2267,4 cd | 192, 5 abc | 10, 7 | 6,01 | 1,89 ab |
| T4: 50%N + *Rp+Pf+Bs+Ba+Bp +Bl+Sb* (500mL/ha) | 7,81 ab | 2305,4 cd | 196,0 ab | 11,05 | 5,94 | 1,87 ab |
| T5: 50%N + *Rp+Pf+Bs+Ba+Bp +Bl+Sb* (1.000mL/ha) | 8,13 a | 2426,8 be | 198,1 ab | 10,28 | 6,01 | 2,17 ab |
| T6: 50%N + *Rp+Pf+Bs+Ba+Bp +Bl+Sb* (2.000mL/ha) | 7,46 ab | 2600,0 ab | 200,0 a | 9,61 | 6,05 | 2,41 a |
| T7: 50%N + *Rp+Pf+Bs+Ba+Bp +Bl+Sb* (3.000mL/ha) | 8,28 a | 2721,0 a | 203,3 a | 9,94 | 6,19 | 2,34 a |
| **CV (%)₃** | **7,78** | **8,86** | **5,36** | **11,78** | **6,05** | **10,02** |

| | | | | | | |
|---|---|---|---|---|---|---|
| 1. Means followed by the same letter in the column, do not present statistical different between the treatments by Duncan test (p≤0,1). 2. ns: non-significant. 3. Coefficient of variation in percentage. Rp: *Rhodopseudomonas palustris;* Sb: *Saccharomyces boulardii;* Bs: *Bacillus subtilis;* Bp: *B. pumilus;* Ba: *B. amyloliquefaciens;* Pf: *P. fluorescens;* Bl: *B. licheniformis.* | | | | | | |

## Claims

1. Soil conditioning and/or decompaction agricultural composition, **characterized by** the fact that it comprises one or more species of the microorganisms *Rhodopseudomonas, Pseudomonas, Saccharomyces, Bacillus,* with addition of chitosan;
wherein the microorganisms comprise the species Rhodopseudomonas palustris, Pseudomonas fluorescens, Bacillus subtilis, B. amyloliquefaciens, B. pumilus, B. licheniformis and Saccharomyces boulardii;
wherein the concentration of each one of the microorganisms can vary in the range comprising 1,0 × 10⁶ to 1,0 × 10⁹ UFC/mL, and the amount of chitosan that composes the product comprises the range between 0,1 to 5% (p/v) of the product.

2. Industrial process for obtaining soil conditioning and/or decompaction agricultural composition, as defined in claim 1, **characterized by** comprising the following steps:
a) fermentation of the microorganisms of different genres, *Rhodopseudomonas, Pseudomonas, Bacillus* and *Saccharomyces,* not being restricted to the cited species;
b) next, after fermentation the mixture of the microorganisms is carried out as well as the packaging of the formulated products.

3. The industrial process, according to claim 2, **characterized by** the fact that the process of production of step (a) occurs in fermentor environment for all species.

4. The industrial process, according to claim 2, **characterized by** the fact that the industrial process of step (a) occurs in fermentor environment of 2.000L, 10.000L and 20.000L, not being restricted solely to these volumes.

5. The industrial process, according to claim 2, **characterized by** the fact that the fermentation of the culture is by batch.

6. The industrial process, according to claim 2, **characterized by** the fact that the process of industrial induction of step (a) is carried out at a temperature of approximately 22 °C to 38 °C.

7. The industrial process, according to claim 2, **characterized by** the fact that the process of industrial induction of step (a) for the species is carried out at an air flow of approximately 3,0 Nm³/h (=0,25 vvm) to approximately 10,0 Nm³/h (=0,83 vvm).

8. The industrial process, according to claim 2, **characterized by** the fact that the process of step (a) further comprises the sequential expansion of the culture of different species of *Rhodopseudomonas, Pseudomonas, Bacillus* and *Saccharomyces* for inoculation of the fermentation culture.

9. The industrial process, according to claim 2, **characterized by** the fact that the species of *Rhodopseudomonas, Pseudomonas, Bacillus* and *Saccharomyces* are inoculated separately.

10. The industrial process, according to claim 2, **characterized by** the fact that the sequential expansion is carried out in volumes of 100 mL, 1L, 10 L, 180 L, 2000 L up to 10.000L.

11. The industrial process, according to claim 10, **characterized by** the fact that the species of *Rhodopseudomonas, Pseudomonas, Bacillus* and *Saccharomyces* are expanded by incubation in orbital agitator at 80 rpm to 200 rpm;
wherein the species of *Rhodopseudomonas, Pseudomonas, Bacillus* and *Saccharomyces* are expanded by incubation for 8 hours to 48 hours;
wherein the species of *Rhodopseudomonas, Pseudomonas* and *Saccharomyces* are expanded in glass flasks, containing 1 L of specific culture medium, and *Bacillus* are expanded in stainless steel flasks containing 1 L of specific culture medium, and 10 L of culture medium in stainless steel flasks;
wherein the species of *Rhodopseudomonas, Pseudomonas, Bacillus* and *Saccharomyces* are incubated at an air flow of 0,25 Nm³/h to 1,0 Nm³/h (=0,41 - 1,67 vvm) for 10 L of culture medium.

12. The industrial process, according to claim 11, **characterized by** the fact that after the segregated cultivation of the species in two stainless steel flasks of 10 L, said two flasks are inoculated in tanks containing 180 L of culture medium;
wherein the strains are incubated for 24 to 168 hours;
wherein the species of *Rhodopseudomonas, Pseudomonas, Bacillus* and *Saccharomyces* are incubated at an air flow of 1,0 to 15,0 Nm³/h (= 0,16 - 1,25 vvm).

13. The industrial process, according to claim 2, **characterized by** the fact that the fermentation step 10.000L of the culture of *Rhodopseudomonas* and fermentation of 2.000L of the culture of *Pseudomonas, Bacillus, Saccharomyces* are carried out at a pressure of 1,0 to 2,0 kgf/cm²;
wherein the fermentation step is carried out with agitation of 40 hz to 45 hz;
wherein the fermentation step is carried out at a temperature of 22 °C to 38 °C;
wherein the fermentation step is carried out at an air flow of 1,0 Nm³/h to 2,5 Nm³/h (= 0,0085 - 0,021 vvm).

14. The industrial process, according to claim 2, **characterized by** the fact that the process of mixture of the product of step (b) is carried out in fermentor of 20.000L, not being restricted solely to this volume, and that step (b) of the mixture of microorganisms in fermentor of 20.000L is conducted for 60 to 180 minutes.

15. Use of the soil conditioning and/or decompaction agricultural composition, as defined in claim 1, **characterized by** the fact that the use thereof is for application in several agricultural cultures;
for application in different dosages and number of applications;
for application in several types of soils;
is also for the soil decompaction;
is also for promoting plant growth;
wherein the application thereof is mainly via furrow, but not being restricted to the use thereof, but also in application via seed or foliar spraying.
